Europäisches Patentamt

European Patent Office

Office européen des brevets

(19)

(11) **EP 1 216 723 B1**

(12) **FASCICULE DE BREVET EUROPEEN**

(45) Date de publication et mention
de la délivrance du brevet:
**02.03.2005 Bulletin 2005/09**

(51) Int Cl.[7]: **A61N 1/37**, G01R 27/26

(21) Numéro de dépôt: **01403332.8**

(22) Date de dépôt: **21.12.2001**

(54) **Dispositif de mesure de l'impédance complexe d'une sonde de dispositif médical implantable actif, notamment de stimulateur cardiaque, défibrillateur et/ou cardioverteur**

Vorrichtung zur Messung der komplexen Impedanz einer Sonde, einer aktiven implantierbaren medizinischen Vorrichtung, insbesondere eines Herzschrittmacher, Defibrillator, und/oder Kardiovertierer

Device for measuring the complex impedance of a probe of an active implantable medical device, in particular of a pacemaker, defibrillator and/or cardioverter

(84) Etats contractants désignés:
**AT BE CH CY DE DK ES FI FR GB GR IE IT LI LU MC NL PT SE TR**

(30) Priorité: **22.12.2000 FR 0016906**

(43) Date de publication de la demande:
**26.06.2002 Bulletin 2002/26**

(73) Titulaire: **ELA MEDICAL**
**92541 Montrouge (FR)**

(72) Inventeur: **Legay, Thierry**
**91640 Fontenay Les Bris (FR)**

(74) Mandataire: **Dupuis-Latour, Dominique et al**
**SEP Pagenberg & Associés**
**14 boulevard Malesherbes**
**75008 Paris (FR)**

(56) Documents cités:
**WO-A-99/58192**          **US-A- 4 683 417**

## Description

L'invention concerne les "dispositifs médicaux implantables actifs" tels que définis par la directive 90/385/CEE du 20 juin 1990 du Conseil des communautés européennes, notamment les dispositifs stimulateurs cardiaques, défibrillateurs et/ou cardioverteurs permettant de délivrer au coeur des impulsions électriques de faible énergie pour le traitement des troubles du rythme cardiaque.

L'invention n'est cependant pas limitée à ces dispositifs particuliers mais s'applique aussi bien, comme on le comprendra, à tout dispositif médical implantable délivrant des stimuli électriques à un tissu par l'intermédiaire d'un conducteur ou d'une sonde.

Ces dispositifs comportent un générateur contenant les divers circuits électroniques et la pile d'alimentation du dispositif. Ce générateur est relié électriquement et mécaniquement à une sonde pourvue d'électrodes de stimulation intracardiaque permettant de détecter les potentiels de dépolarisation du myocarde et délivrer à ce dernier, en tant que de besoin, les impulsions de stimulation produites par le générateur.

Un paramètre important d'une sonde implantée est son impédance, car ce paramètre conditionne le courant nécessaire à la stimulation (d'autant plus faible que l'impédance à l'interface coeur/électrode est élevée) et, par voie de conséquence, la durée de vie du stimulateur.

Cette impédance est un paramètre qui peut évoluer au cours du temps, car il dépend non seulement des caractéristiques intrinsèques de la sonde (géométrie, matériaux, etc.) mais aussi des caractéristiques électriques de l'interface sonde/myocarde, qui peuvent évoluer au cours du temps pour diverses raisons, en particulier l'évolution de l'environnement de la tête de sonde (formation de tissu réactionnel de contact) et l'altération du matériau conducteur formant l'électrode de la sonde.

Il importe donc de pouvoir mesurer régulièrement l'impédance de la sonde, pour voir si cette impédance reste dans des limites acceptables et réajuster éventuellement les paramètres électriques de délivrance de l'impulsion de stimulation en fonction de la valeur ainsi mesurée.

Cette mesure est rendue difficile par le fait que l'impédance de la sonde est une impédance complexe, comprenant une composante résistive pure et une composante capacitive, composantes qui peuvent varier chacune de manière différente au cours du temps.

L'impédance de sonde $Z_s$ peut être modélisée par une résistance pure $R_s$ en série avec une capacité $C_h$ appelée "capacité de Helmholtz". La composante résistive est seule responsable du courant consommé à chaque stimulus, tandis la composante capacitive est, pour sa part, responsable de la perte par polarisation à l'interface coeur/électrode.

La plupart des prothèses actuelles opèrent une évaluation de l'impédance complexe qui amalgame $R_s$ et $C_h$, et qui se trouve au surplus être très imprécise et variable en fonction du signal de mesure utilisé : la valeur du résultat fourni par ces appareils varie notamment selon la durée de l'impulsion électrique de stimulation,.

Le WO-A-99/58192 propose un procédé permettant de déterminer isolément les composantes résistives et capacitives pour pallier ces inconvénients, mais il nécessite une circuiterie complexe, mettant notamment en oeuvre une résistance shunt dans le circuit de stimulation.

L'un des buts de l'invention est de proposer un dispositif permettant la mesure la composante résistive d'une sonde de prothèse implantable indépendamment de la composante capacitive, et ceci de manière exacte et reproductible, permettant ainsi de déterminer avec toute la précision voulue le courant nécessaire à la stimulation et, corrélativement, évaluer la durée de vie probable de la prothèse.

Comme on le verra, l'invention permet également, si on le souhaite, la détermination de la composante capacitive de l'impédance de sonde (capacité de Helmholtz), indépendamment de la composante résistive. Ce paramètre permet notamment d'évaluer la perte par polarisation, problème qui réapparaît en effet avec les électrodes à très faible surface (de l'ordre de 1,5 mm$^2$ ou moins), après avoir été résolu en donnant aux électrodes distales une structure granuleuse procurant de fortes capacités de Helmholtz dont on pouvait s'affranchir en pratique.

À cet effet, le dipositif de l'invention comprend des moyens aptes à: produire une impulsion de stimulation par décharge dans la sonde d'une capacité-réservoir du dispositif, préalablement amenée à un niveau de tension donné ; mesurer la variation de la tension aux bornes de la capacité-réservoir pendant la durée de la décharge ; et déterminer l'impédance de sonde à partir de la tension ainsi mesurée.

De façon caractéristique de l'invention, ladite mesure comprend l'échantillonnage d'au moins trois valeurs successives de la tension aux bornes de la capacité-réservoir, et ladite détermination comprend la détermination séparée des composantes résistive et/ou capacitive de l'impédance de la sonde à partir desdites au moins trois valeurs de tension échantillonnées ainsi obtenues.

Très avantageusement, ladite détermination est opérée par calcul algébrique, notamment en opérant l'échantillonnage à des instants choisis de manière que le troisième temps d'échantillonnage corresponde à une durée double du deuxième temps d'échantillonnage, le premier temps d'échantillonnage étant pris au début de l'impulsion de stimulation.

Dans un premier mode de mise en oeuvre, lesdites au moins trois valeurs successives sont échantillonnées au cours de la même impulsion de stimulation.

Dans un second mode de mise en oeuvre, lesdites au moins trois valeurs successives sont échantillonnées au cours d'au moins deux impulsions de stimulation successives, de préférence produites de sorte que l'une soit de durée double de l'autre, les instants d'échantillonnage étant choisis au début et à la fin de chacune des deux impulsions.

On va maintenant décrire un exemple de mise en oeuvre de l'invention, en référence aux dessins annexés.

La figure 1 est une vue schématique des divers éléments et circuits impliqués dans la mesure de l'impédance de sonde selon l'invention.

La figure 2 illustre le profil de l'impulsion de stimulation permettant la mesure d'impédance selon un premier mode de mise en oeuvre de l'invention.

La figure 3 illustre le profil des impulsions de stimulation utilisées dans un second mode de mise en oeuvre de l'invention.

Comme on l'a indiqué au début de la description, du point de vue électrique la sonde 10 peut être modélisée par une impédance $Z_s$ constituée d'une résistance pure $R_s$ en série avec une capacité de liaison $C_h$ dite capacité de Helmholtz.

Par convention, cette impédance de sonde $Z_s$ englobe aussi celle du conducteur reliant la sonde proprement dite au générateur 20, notamment les résistances ohmiques de circuiterie en sus des résistances des tissus.

Le circuit de stimulation comprend, essentiellement, une capacité-réservoir 22 de valeur C, un commutateur 24 piloté par un signal logique s(t) et une capacité de liaison 26 de valeur C'.

La capacité C est chargée par un circuit approprié, non représenté (classique en lui-même). La fermeture du commutateur 24 provoque la décharge de cette capacité et par voie de conséquence l'envoi de l'impulsion électrique de dépolarisation à la sonde et au myocarde.

La mesure d'impédance est opérée par mesure de la tension V(t) aux bornes de la capacité C. Cette mesure est réalisée par un diviseur-échantillonneur-bloqueur 28 et un convertisseur analogique/numérique 30 apte à délivrer une valeur numérisée de tension au microcontrôleur 32. Le circuit 28 est conçu pour modifier le rapport de division en fonction de l'amplitude de la tension stockée dans la capacité C, de manière à ramener la tension divisée dans la gamme de conversion du convertisseur 30.

Le microcontrôleur 32 assure la mémorisation des valeurs de tension mesurées, ainsi que les divers calculs. En variante, notamment si les calculs sont trop complexes et/ou ou trop longs, le microcontrôleur peut se contenter de transmettre par télémétrie les valeurs mesurées et mémorisées à un programmateur externe, qui effectuera alors les calculs requis.

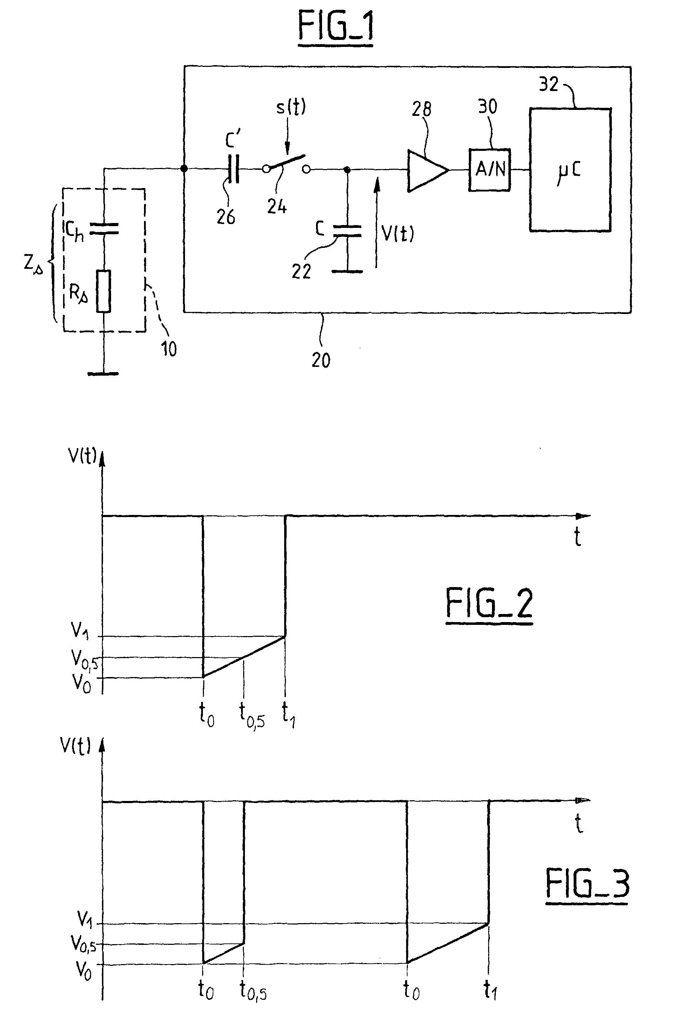

L'impulsion de stimulation présente l'allure générale illustrée figure 2.

Lorsqu'à l'instant $t_0$ le commutateur 24 est fermé par la commande logique s(t), la capacité-réservoir C se décharge dans un circuit comprenant, en série, le commutateur 24, la capacité de liaison C', la capacité de Helmholtz $C_h$ et la résistance $R_s$ (les résistances série du commutateur et des conducteurs de routage, typiquement de quelques ohms ou quelques dizaines d'ohms au plus, sont prises en compte dans $R_s$ car négligeables devant cette dernière : moins de 1 % de la valeur $R_s$).

La tension V(t) aux bornes de la capacité-réservoir C décroît, à partir de l'instant $t_0$ et jusqu'à l'instant $t_1$ de réouverture du commutateur 24, suivant une loi exponentielle, classique des réseaux RC, donnée par :

$$V(t) = \frac{V_0 * [C + C_s*\exp\{-t/R_s*(1/C + 1/C_s)\}]}{C + C_s}$$

avec $C_s = 1 / (1/C' + 1/C_h)$, $V_0$ étant la tension initiale aux bornes de C.

De façon caractéristique de l'invention, la tension V(t) est échantillonnée puis convertie en numérique de façon à obtenir trois valeurs de tension consécutives : $V_0$ à l'instant $t_0$, $V_{0,5}$ à un instant $t_{0,5}$ à mi-durée d'impulsion, et $V_1$ à l'instant $t_1$ correspondant à la fin de l'impulsion.

À partir de ces valeurs, il est possible par un calcul algébrique d'obtenir les valeurs des composantes de l'impédance de sonde, données par :

$$R_s = \frac{-V_0 * t_1 * (V_0 - 2*V_{0.5} + V_1)}{2 * C * L_n[(V_0-V_1)/(V_0-V_{0,5})-1] * (V_0-V_{0,5})^2}$$

et

$$C_h = \frac{C * C'}{C' * [((V_0*V_1) - V_{0,5}^2) /(V_0-V_{0,5})^2]- C}$$

l'instant $t_0$ étant choisi comme origine des temps.

On notera que le choix, pour le troisième temps d'échantillonnage ($t_1$), d'une durée égale au double de la durée du second temps d'échantillonnage ($t_{0,5}$) permet de donner une solution analytique au calcul et évite ainsi un calcul itératif, qui serait sinon très lourd à mettre en oeuvre compte tenu des ressources limitées en terme de puissance de calcul, que ce soit au sein de l'implant ou même par un programmateur.

Une variante de mise en oeuvre de ce procédé est illustrée figure 3.

Au lieu d'effectuer le triple échantillonnage au cours d'une seule et même impulsion de stimulation, il est possible d'utiliser deux impulsions de stimulation consécutives, de même amplitude et de durée double l'une de l'autre, en échantillonnant pour chacune d'elles la valeur de tension aux bornes de la capacité-réservoir C au début et à la fin des impulsions respectives. De préférence, l'impulsion la plus courte est la première, car la décharge du condensateur C est ainsi moindre.

La mise en oeuvre du procédé par cette variante est certes un peu moins précise, car elle présuppose que la tension de stimulation initiale $V_0$ soit exactement la même pour les deux impulsions. Elle est toutefois plus simple à mettre en oeuvre et ne nécessite pas des moyens de calcul aussi rapides que la première méthode.

Enfin, tout ce qui a été décrit précédemment l'a été dans le contexte d'une configuration unipolaire, mais s'applique bien entendu aussi, *mutatis mutandis,* à une configuration bipolaire. On notera que, dans une configuration bipolaire, la capacité modélisée $C_h$ est la somme de capacités correspondant aux deux électrodes, proximale et distale, et que la capacité de l'électrode proximale est très supérieure à celle de l'électrode distale, compte tenu de la forme annulaire, de plus grande surface, de cette électrode proximale.

## Revendications

1. Un dispositif de mesure de l'impédance complexe d'une sonde de dispositif médical implantable actif, notamment de dispositif stimulateur cardiaque, défibrillateur et/ou cardioverteur, dispositif comprenant des moyens aptes à :

   - produire une impulsion de stimulation par décharge dans la sonde (10) d'une capacité-réservoir (22) du dispositif (20), préalablement amenée à un niveau de tension donné,
   - mesurer la variation de la tension (V(t)) aux bornes de la capacité-réservoir pendant la durée de la décharge, et
   - déterminer l'impédance de sonde ($Z_s$) à partir de la tension ainsi mesurée,

   dispositif **caractérisé en ce que** :

   - ladite mesure comprend l'échantillonnage d'au moins trois valeurs successives ($V_0$, $V_{0,5}$, $V_1$) de la tension aux bornes de la capacité-réservoir, et
   - ladite détermination comprend la détermination séparée des composantes résistive ($R_s$) et/ou capacitive ($C_h$) de l'impédance de la sonde à partir desdites au moins trois valeurs de tension échantillonnées ainsi obtenues.

2. Le dispositif de la revendication 1, dans lequel ladite détermination est opérée par calcul algébrique.

3. Le dispositif de la revendication 1, dans lequel ledit échantillonnage est opéré à des instants ($t_0$, $t_{0,5}$, $t_1$) choisis de manière que le troisième temps d'échantillonnage ($t_1$) corresponde à une durée double du deuxième temps d'échantillonnage ($t_{0,5}$), le premier temps d'échantillonnage ($t_0$) étant pris au début de l'impulsion de stimulation.

4. Le dispositif de la revendication 1, dans lequel lesdites au moins trois valeurs successives ($V_0$, $V_{0,5}$, $V_1$) sont échantillonnées au cours de la même impulsion de stimulation.

5. Le dispositif de la revendication 1, dans lequel lesdites au moins trois valeurs successives ($V_0$, $V_{0,5}$, $V_1$) sont échantillonnées au cours d'au moins deux impulsions de stimulation successives.

6. Le dispositif de la revendication 5, dans lequel lesdites deux impulsions successives de stimulation sont produites de sorte que l'une soit de durée double de l'autre, les instants d'échantillonnage étant choisis au début ($t_0$, $t_0$) et à la fin ($t_{0,5}$, $t_1$) de chacune des deux impulsions.

## Patentansprüche

1. Vorrichtung zur Messung der komplexen Impedanz einer Sonde einer aktiven implantierbaren medizinischen Vorrichtung, insbesondere eines Herzschrittmachers, Defibrillators und/oder Kardiovertierers, wobei die Vorrichtung Mittel umfasst, die geeignet sind:

   - einen Stimulationsimpuls zu produzieren durch Entladen in der Sonde (10) einer Vorratskapazität (22) der Vorrichtung (20), die zuvor auf ein gegebenes Spannungsniveau geführt wurde,
   - Messen der Spannungsänderung (V(t)) an den Anschlüssen der Vorratskapazität während der Dauer der Entladung, und
   - Bestimmen der Impedanz der Sonde ($Z_s$) ausgehend von der so gemessenen Spannung,

   wobei die Vorrichtung **dadurch gekennzeichnet ist, dass**:

   - die Messung das Abtasten mindestens zweier aufeinanderfolgender Werte ($V_0$, $V_{0,5}$, $V_1$) der Spannung an den Anschlüssen der Vorratskapazität umfasst, und
   - die Bestimmung die getrennte Bestimmung der resistiven ($R_s$) und/oder kapazitiven ($C_h$) Bestandteile der Impedanz der Sonde ausgehend von den so erhaltenen abgetasteten mindestens drei Spannungswerten umfasst.

2. Vorrichtung nach Anspruch 1, in welcher die Bestimmung ausgeführt wird durch eine algebraische Berechnung.

**3.** Vorrichtung nach Anspruch 1, in welcher das Abtasten ausgeführt wird zu Zeitpunkten ($t_0$, $t_{0,5}$, $t_1$), derart ausgewählt, dass der dritte Abtastzeitpunkt ($t_1$) einer doppelten Dauer des zweiten Abtastzeitpunkts ($t_{0,5}$) entspricht, wobei der erste Abtastzeitpunkt ($t_0$) am Anfang des Stimulationsimpulses genommen wird.

**4.** Vorrichtung nach Anspruch 1, in welcher die mindestens drei aufeinanderfolgenden Werte ($V_0$, $V_{0,5}$, $V_1$) abgetastet werden im Verlauf des gleichen Stimulationsimpulses.

**5.** Vorrichtung nach Anspruch 1, in welcher die mindestens drei aufeinanderfolgenden Werte ($V_0$, $V_{0,5}$, $V_1$) abgetastet werden im Verlauf von mindestens zwei aufeinanderfolgenden Stimulationsimpulsen.

**6.** Vorrichtung nach Anspruch 5, in welcher die zwei aufeinanderfolgenden Stimulationsimpulse so produziert werden, dass einer von doppelter Dauer des anderen ist, wobei die Abtastzeitpunkte ausgewählt sind am Anfang ($t_0$, $t_0$) und am Ende ($t_{0,5}$, $t_1$) jedes der beiden Impulse.

**Claims**

**1.** A device for measuring the complex impedance of a lead of an active implantable medical device, in particular of a cardiac pacemaker, defibrillator and/or cardiovertor, said device comprising means adapted for:

- producing a stimulation pulse by discharging to the lead (10) a tank-capacitor (22) of the device (20), previously brought to a given voltage level;
- measuring the voltage variation ($V(t)$) at the terminals of the tank-cap-acitor for the duration of the discharge; and
- determining the lead impedance ($Z_s$) from the voltage thus measured,

said device being **characterised in that**:

- said measuring comprises sampling at least three successive values ($V_0$, $V_{0,5}$, $V_1$) of the voltage at the terminals of the tank capacitor, and
- said determining comprises separately determining the resistive ($R_s$) and/or capacitive ($C_h$) components of the impedance of the lead from said at least three sampled voltage value thus obtained.

**2.** The device of claim 1, wherein said determining is performed by an algebraic calculation.

**3.** The device of claim 1, wherein said sampling is performed on times ($t_0$, $t_{0,5}$, $t_1$) which are selected so that the third sampling time ($t_1$) correspond to twice the duration of the second sampling time ($t_{0,5}$), the first sampling time ($t_0$) being taken at the beginning of the stimulation pulse.

**4.** The device of claim 1, wherein said at least three successive values ($V_0$, $V_{0,5}$, $V_1$) are sampled during the same stimulation pulse.

**5.** The device of claim 1, wherein said at least three successive values ($V_0$, $V_{0,5}$, $V_1$) are sampled during at least two successive stimulation pulses.

**6.** The device of claim 5, wherein said two successive stimulation pulses are so produced that one have a duration twice the other one, the sampling times being selected at the beginning ($t_0$, $t_0$) and at the end ($t_{0,5}$, $t_1$) of each of said pulses.

## FIG_1

## FIG_2

## FIG_3